# EUROPÄISCHE PATENTANMELDUNG

(11) **EP 2 902 074 A1**
(43) Veröffentlichungstag der Anmeldung: **05.08.2015**
(21) Anmeldenummer: 15150321.6
(22) Anmeldetag: 07.01.2015
(51) Int. Cl.: A61N 1/05

(54) **ELEKTRODENFIXIERHÜLSE MIT VERSTEIFTEM BEFESTIGUNGSABSCHNITT UND VERFAHREN ZU DEREN HERSTELLUNG**

(30) Priorität: 29.01.2014 US 201461932804 P
(71) Anmelder: BIOTRONIK SE & Co. KG, 12359 Berlin (DE)
(72) Erfinder: Farhat, Chiheb, 14169 Berlin (DE); Voigt, Siegfried, 12161 Berlin (DE); Grosse, Ines, 10247 Berlin (DE)
(74) Vertreter: Galander, Marcus

(57) **Zusammenfassung**

Die Erfindung betrifft eine Elektrodenfixierhülse (1) zur Fixierung einer Elektrode an einem Gewebeabschnitt eines Patienten, sowie ein Verfahren zu deren Herstellung. Um eine Beschädigung der Elektrode durch deren Befestigung an der Elektrodenfixierhülse (1) zu vermeiden, ist erfindungsgemäß vorgesehen, dass der Befestigungsabschnitt (2) mit einer größeren Steifigkeit ausgebildet wird als ein benachbarter Abschnitt (3, 4) der Elektrodenfixierhülse (1).

## Beschreibung

Die Erfindung betrifft eine Elektrodenfixierhülse zur Fixierung einer Elektrode an einem Gewebeabschnitt eines Patienten, mit einem Befestigungsabschnitt zur Befestigung der Elektrodenfixierhülse an der Elektrode, wobei die Elektrodenfixierhülse eine sich in ihrer Längsrichtung durch den Befestigungsabschnitt erstreckende Aufnahmeröhre für die Elektrode ausbildet und entgegen einer senkrecht zur Längsrichtung verlaufende Radialrichtung zusammendrückbar ausgebildet ist. Ferner betrifft die Erfindung ein Verfahren zum Herstellen einer Elektrodenfixierhülse zur Fixierung einer Elektrode an einem Gewebeabschnitt eines Patienten, mit einem Befestigungsabschnitt zur Befestigung der Elektrodenfixierhülse an der Elektrode, wobei die Elektrodenfixierhülse mit einer sich in ihrer Längsrichtung durch den Befestigungsabschnitt erstreckenden Aufnahmeröhre für die Elektrode und der Befestigungsabschnitt entgegen einer senkrecht zur Längsrichtung verlaufenden Radialrichtung zusammendrückbar ausgeformt wird.

Elektrodenfixierhülsen und Verfahren zu deren Herstellung sind allgemein bekannt. Elektrodenfixierhülsen sind leicht verformbar, sodass diese zumindest teilweise in Gefäße eingesetzt werden können, ohne die Gefäße zu beschädigen. Bei bekannten Elektrodenfixierhülsen besteht jedoch das Problem, dass die durch die Elektrodenfixierhülse geführte Elektrode beschädigt werden kann, wenn durch eine Befestigungskraft zur Befestigung der Elektroden-fixierhülse an der Elektrode ein zu großer Druck auf die Elektrodenfixierhülse ausgeübt wird. Schutzhülsen, welche beim Implantieren der Elektrodenfixierhülse zwischen der Elektrodenfixierhülse und der Elektrode zum Schutz der Elektrode vor mechanischer Überlastung anzuordnen sind, erschweren das Einsetzen und Befestigen der Elektrodenfixierhülse, sodass ein die Elektrodenfixierhülse einsetzender Arzt einen erhöhten Aufwand leisten muss.

Daher liegt der Erfindung die Aufgabe zugrunde, eine Elektrodenfixierhülse und ein Verfahren zu deren Herstellung bereitzustellen, wobei die Elektrodenfixierhülse einfach implantierbar und die Elektrode vor Beschädigungen geschützt ist.

Für die eingangs genannte Elektrodenfixierhülse ist die Aufgabe erfindungsgemäß dadurch gelöst, dass der Befestigungsabschnitt eine größere Steifigkeit aufweist, als ein benachbarter Abschnitt der Elektrodenfixierhülse. Für das eingangs genannte Verfahren ist die Aufgabe dadurch gelöst, dass der Befestigungsabschnitt mit einer größeren Steifigkeit ausgeformt wird, als ein benachbarter Abschnitt der Elektrodenfixierhülse.

Durch die höhere Steifigkeit des Befestigungsabschnitts im Vergleich zum benachbarten Abschnitt, zum Beispiel ein Führungsabschnitt, der beispielsweise in ein Gefäß eingesetzt werden kann, werden lokale Überlastungen der Elektrode, wie sie beispielsweise durch Verwendung von Ligaturfäden, die durch zusammenziehen einen inneren Durchmesser der Aufnahmeröhre zu verringern und hierdurch die Elektrodenfixierhülse an der Elektrode zu befestigen suchen, sicher verhindert. Der steifere Befestigungsabschnitt verteilt die Befestigungskräfte und verhindert insbesondere punkt- oder linienförmige Überlastungen. Da die Elektrodenfixierhülse außerhalb des Befestigungsabschnittes weiterhin eine höhere Flexibilität aufweisen kann als im Bereich des Befestigungsabschnitts kann die Elektrodenfixierhülse ohne Weiteres wie bisher in Gefäße des Patienten eingesetzt werden, ohne das die Gefäße hierdurch geschädigt werden.

Die erfindungsgemäße Lösung kann durch verschiedene, jeweils für sich vorteilhafte, beliebig miteinander kombinierbare Ausgestaltung weiter verbessert werden. Auf diese Ausgestaltungsformen und die mit Ihnen verbundenen Vorteile ist im Folgenden eingegangen.

So kann ein Versteifungselement in den Befestigungsabschnitt integriert werden. Insbesondere kann das Versteifungselement unverlierbar mit einem Wandmaterial des Befestigungsabschnitts verbunden werden. Die Elektrodenfixierhülse kann also das Versteifungselement aufweisen, wobei das Versteifungselement im Befestigungsabschnitt angeordnet oder sogar befestigt ist und eine größere Steifigkeit aufweist, als das Wandmaterial der Elektrodenfixierhülse. Dabei kann das Versteifungselement ein integraler Bestandteil der Elektrodenfixierhülse sein. Das Versteifungselement ist unverlierbar in der Elektrodenfixierhülse gehalten und in diese integriert, sodass das Versteifungselement beim Implantieren der Elektrodenfixierhülse nicht separat gehandhabt werden braucht. Auch kann das Versteifungselement nicht beim Einsetzen oder Durchführen der Elektrode durch die Aufnahmeröhre verrutschen, wenn das Versteifungselement unverschieblich beziehungsweise unverlierbar am Wandmaterial befestigt ist. Die Elektrodenfixierhülse ist also zusammen mit dem Versteifungselement einstückig handhabbar ausgebildet, sodass das Versteifungselement beim Einsetzen der Elektrode nicht separat gehandhabt oder gesichert werden muss.

Um die Elektrode im Bereich des Befestigungsabschnitts in der Elektrodenfixierhülse befestigen zu können, kann das Versteifungselement entgegen in der Radialrichtung verformbar und insbesondere zusammendrückbar sein. Beispielsweise durch den Ligaturfaden aufbringbare Befestigungskräfte können das Versteifungselement verformen oder zusammendrücken, sodass das Versteifungselement die durch den Ligaturfaden aufgebrachten Befestigungskräfte an die Elektrode verteilt weitergeben kann. Die Elektrode kann daher kraftschlüssig in der Aufnahmeröhre gehalten sein. Das Versteifungselement verteilt dabei die zum Beispiel linienförmig aufgebrachten Befestigungskräfte des Ligaturfadens so, dass lokale mechanische Überlastungen der Elektrode verhindert werden.

Damit das Versteifungselement insbesondere entlang der Längsrichtung unverschieblich durch das Wandmaterial der Elektrodenfixierhülse gehalten ist, kann das Versteifungselement in das Wandmaterial des Befestigungsabschnitts insbesondere in der Radialrichtung eingebettet werden. Dadurch, dass das Versteifungselement in das Wandmaterial insbesondere in der Radialrichtung eingebettet oder eingelassen ist, ist es wenigstens in und womöglich auch entgegen der Längsrichtung unverschieblich durch das Wandmaterial gehalten, sodass Relativbewegungen der Elektrode entlang der Längsrichtung durch die Aufnahmeröhre nicht zu einer Verschiebung des Versteifungselements relativ zum Wandmaterial und ebenfalls entlang der Längsrichtung führen können.

Das Versteifungselement kann an ein durch die Aufnahmeröhre begrenztes oder definiertes Aufnahmevolumen für die Elektrode angrenzen. Das Versteifungselement kann also direkt an der Elektrode anliegen und diese somit sicher mechanisch kontaktieren und halten. Um zu gewährleisten, dass die Elektrode entlang der Längsrichtung einfach durch die Aufnahmeröhre gleiten kann, kann eine Innenseite des Versteifungselements zumindest im nicht zusammengedrückten Zustand der Elektrodenfixierhülse mit einer Innenseite des Befestigungsabschnitts fluchten. Die Aufnahmeröhre kann also im Wesentlichen ohne Stufe beim Übergang von Wandmaterial zum Versteifungselement begrenzt sein. Eine Kollision der Elektrode mit einem vorspringenden Abschnitt des Versteifungselements oder des Wandmaterials ist somit sicher verhindert, sodass die Elektrode einfach in die Aufnahmeröhre einsetzbar ist.

Gemäß einer besonders vorteilhaften Ausgestaltungsform kann das Versteifungselement als eine Hohlwendel ausgebildet sein, wobei sich das Versteifungselement um das Aufnahmevolumen für die Elektrode herumwindet und eine zentrale Achse der Hohlwendel parallel zur Längsrichtung ausgerichtet ist. Durch die Ausbildung des Versteifungselements als Hohlwendel ist die Elektrode im Bereich des Befestigungsabschnitts allseitig vor Überlastungen geschützt, da diese im Bereich des Befestigungsabschnitts von der Hohlwendel umgeben ist. Auch wenn einzelne Windungen der Hohlwendel zumindest im nicht zusammengedrückten Zustand des Befestigungsabschnitts nicht aneinander anliegen und somit die Elektrode dicht umgeben, verteilt die Hohlwendel Befestigungskräfte so, dass linienförmige Belastungen verteilt an die Elektrode abgegeben werden.

Werden zur Befestigung der Elektrodenfixierhülse an der Elektrode die entgegen der Radialrichtung wirkenden Befestigungskräfte auf den Befestigungsabschnitt ausgeübt, so kann ein Innendurchmesser der Hohlwendel dadurch verringert sein. Die Hohlwendel kann nämlich durch die Befestigungskräfte so verformbar sein, dass sich ihr Innendurchmesser ändert, insbesondere gleichmäßig entlang der Längsrichtung. Die Hohlwendel ist vorzugsweise elastisch entgegen Radialrichtung verformbar. Soll die Elektrode beispielsweise aus der Elektroden-fixierhülse wieder entnommen werden, so reicht es aus, die Befestigungskräfte zu reduzieren und beispielsweise den Ligaturfaden zu lockern oder zu entfernen. Eine elastische Rückstellkraft der Hohlwendel weitet deren Innendurchmesser, wenn die Befestigungskräfte zu gering sind, sodass die Elektrode einfach durch die Aufnahmeröhre herausgezogen werden kann. Alternativ oder zusätzlich kann das Wandmaterial elastisch entgegen der Radialrichtung verformbar sein und die Rückstellkraft aufbringen oder dazu beitragen. Zur zumindest teilweisen Übertragung der Rückstellkraft von Wandmaterial an das Versteifungselement haftet das Versteifungselement vorzugsweise am Wandmaterial.

Die Hohlwendel kann eine gewundene Versteifungsplatte aufweisen oder aus einer solchen bestehen, wobei parallel zur Längsrichtung verlaufende Seiten der Versteifungsplatte größer sind, als quer zur Längsrichtung verlaufende Seiten der Versteifungsplatte. Die Versteifungsplatte kann also streifenförmig ausgebildet sein, wobei sich die Versteifungsplatte um die zentrale Achse der Hohlwendel herum windet. Das Größenverhältnis der Seiten der Versteifungsplatte gewährleistet, dass die Versteifungsplatte in der Radialrichtung wenig Raum beansprucht. Ferner kann die Hohlwendel aufgrund der großen parallel zur Längsrichtung verlaufenden Seiten möglichst großflächig an der Elektrode anliegen, sodass die Befestigungskräfte optimal verteilt werden können. Die Hohlwendel kann mit verschiedenen Querschnitten, zum Beispiel rechteckig oder oval, ausgeführt sein.

Ein parallel zur Längsrichtung bemessener Abstand benachbarter Windungen der Hohlwendel ist vorzugsweise kleiner, als eine parallel zur Längsrichtung bemessene Breite der Versteifungsplatte. Dadurch, dass die Windungen nicht aneinander anliegen, ist eine einfache Verformung der Hohlwendel entlang der Radialrichtung gewährleistet. Würden die Windungen aneinander anliegen, wäre die Verformbarkeit des als Hohlwendel ausgebildeten Versteifungselements deutlich verschlechtert. Der Vergleich zur Breite kleine Abstand gewährleistet, dass die Befestigungskräfte nicht an der Versteifungsplatte vorbei die Elektrode überlasten.

Um die Elektrode quer zur Längsrichtung allseitig gegen Überlastungen schützen zu können, kann die Hohlwendel wenigstens eine Windung aufweisen. Weist die Hohlwendel weniger als eine Windung auf, so ist in einer Umfangsrichtung der Elektrodenfixierhülse zumindest ein Abschnitt der Elektrode für punkt- oder linienförmige Belastungen durch Befestigungskräfte, zum Beispiel hervorgerufen durch den Ligaturfaden, erreichbar und kann hierdurch mechanisch überlastet werden.

Die Hohlwendel kann ferner mehr als eine Windung aufweisen, damit entlang der Längsrichtung ein größerer Abschnitt der Aufnahmeröhre von der Hohlwendel umgeben sein kann. Hierdurch ist die Elektrode entlang der Längsrichtung großflächiger vor Überlastungen schützbar und die Befestigungskräfte können über eine größere Fläche verteilt werden.

Vorzugsweise weist die unverformte Hohlwendel eine ganzzahlige Anzahl von Windungen auf. Beispielsweise kann die Hohlwendel 2, 3, 4, 5, 7 oder bis zu 10 beziehungsweise noch mehr Windungen aufweisen. Entlang der Längsrichtung kann eine Hohlwendel mit einer ganzzahligen Anzahl von Windungen auftretende Befestigungskräfte insbesondere an in und entgegen der Längsrichtung liegenden Enden der Hohlwendel gleichmäßiger verteilen als eine Hohlwendel mit einer nichtganzzahligen Anzahl an Windungen.

Die Hohlwendel ist beispielsweise als eine linksgängige oder als eine rechtsgängige Helix ausgebildet, die sich mit konstanter Steigung um die Aufnahmeröhre windet. Die Steigung der Hohlwendel kann gering sein, um möglichst viele Windungen im Befestigungsabschnitt anordnen zu können. Insbesondere kann ein Winkel zwischen der Längsrichtung und den quer zur Längsrichtung verlaufenden Seiten der Versteifungsplatte größer als 45° sein und beispielsweise 60°, 70°, 80° oder sogar 85° betragen.

Durch die Verwendung des hohlwendelförmigen Versteifungselementes werden jedoch nicht nur mechanische Überlastungen der Elektrode sicher verhindert. Insbesondere die quer zur Längsrichtung verlaufenden Seiten können eine Innere Dichtstrecke der Elektrodenfixierhülse so vergrößern, dass in die Elektrodenfixierhülse eindringende Fluide nicht oder nur verzögert die Elektrodenfixierhülse passieren können.

Alternativ zur hohlwendelförmigen Ausgestaltung des Versteifungselementes kann dieses auch als eine entlang der Längsrichtung geschnittene und quer zur Längsrichtung aufklappbare Versteifungshülse ausgebildet sein. Im zusammengeklappten Zustand der Versteifungshülse kann diese zylinderförmig ausgeformt sein, wobei sich eine Höhenrichtung des Zylinders parallel zur Längsrichtung erstreckt. Zum Aufklappen der Versteifungshülse kann diese zwei Teilschalen aufweisen, die relativ zueinander beweglich miteinander verbunden sind. Beispielsweise können die Teilschalen über ein Scharnier miteinander verbunden sein. Um ein ungewolltes Öffnen der Versteifungshülse zu verhindern, kann diese eine Rastvorrichtung aufweisen, welche die Teilschalen in einer geschlossenen Position halten kann. Zum Öffnen der verrasteten Teilschalen kann eine Rasteinrichtung einer der Teilschalen aus einer Rastposition elastisch herausgedrückt werden. Dabei kann die Versteifungshülse selbst Öffnungskräfte hervorrufen, welche die Teilschalen zum Öffnen der Versteifungshülse bewegen, sobald die Rastverbindung zwischen den Teilschalen gelöst ist. Alternativ oder zusätzlich kann das Wandmaterial der Elektrodenfixierhülse bei der geschlossenen Versteifungshülse elastisch verformt sein, sodass das Wandmaterial die Aufnahmehülse öffnet, sobald die Rastverbindung zwischen den Teilschalen gelöst ist.

Beispielsweise können die Rasteinrichtungen der Teilschalen unterschiedlich ausgebildet sein. Insbesondere können die Rasteinrichtung einer der Teilschalen an einer Innenfläche und die Rasteinrichtung der anderen der Teilschalen an einer Außenfläche der jeweiligen Teilschale angeordnet sein.

Gemäß einer weiteren möglichen Ausgestaltungsform kann das Versteifungselement als ein dickwandiger Versteifungs- oder Aufnahmezylinder ausgebildet sein, dessen Außendurchmesser deutlich größer sein kann, als andere Abschnitte der Elektrodenfixierhülse und insbesondere als ein Außendurchmesser des Führungsabschnittes der Elektrodenfixierhülse. Damit der dickwandige Aufnahmezylinder in die Elektrodenfixierhülse eingesetzt werden kann, kann diese einen Aufnahmebehälter ausbilden, wobei zwei Teilschalen des Aufnahmebehälters parallel zur Längsrichtung auf den Aufnahmezylinder aufgesetzt werden können.

Eine weitere alternative Möglichkeit zur Ausbildung des Versteifungselementes ist, dass Versteifungselement zylinderförmig auszubilden. Das zylinderförmige Versteifungselement ist jedoch oftmals nur schwer oder mit den zur Verfügung stehenden Befestigungskräften nicht in der Radialrichtung verformbar. Damit die Elektrode in der Elektrodenfixierhülse befestigt werden kann, kann die Elektrodenfixierhülse ein zusätzlich zur Elektrode in das zylinderförmige Versteifungselement einsetzbares Fixierelement aufweisen, das die Elektrode im Versteifungselement verpresst. Das Fixierelement kann beispielsweise entlang der Längsrichtung in das zylinderförmige Versteifungselement eingesetzt werden. Alternativ kann das Fixierelement entgegen der Radialrichtung in das Versteifungselement eingesetzt werden.

Darüberhinaus kann anstelle der Verwendung von Ligaturfäden eine Klemmeinrichtung bereitgestellt werden, welche keine linien- oder punktförmigen, sondern großflächige Befestigungskräfte aufbringt. Die Klemmeinrichtung weist vorzugsweise einen im Wesentlichen zylinderförmigen Klemmabschnitt auf, der entlang der Längsrichtung geschnitten und quer zur Längsrichtung elastisch verformbar ist. Zum Aufbringen der Befestigungskräfte kann die Klemmeinrichtung zwei Kraftaufnahmeabschnitte aufweisen, die in der Radialrichtung von entlang der Umfangsrichtung einander gegenüberliegenden Seiten des Kraftaufnahmeabschnitts vorspringen. Beispielsweise können die Kraftaufnahmeabschnitte Öffnungen zur Aufnahme einer Schraube aufweisen, wobei die Schraube die Befestigungskraft in die Kraftaufnahmeabschnitte einleitet, von wo diese durch den Klemmabschnitt an den Kraftaufnahmeabschnitt der Elektrodenfixierhülse oder direkt an die Elektrode geleitet werden.

Das Versteifungselement kann aus verschiedenen Materialien hergestellt werden, beispielsweise aus einem Metall, Polymer oder Thermoplast. Die Oberfläche des Versteifungselements kann zusätzlich mit verschiedenen Oberflächenverfahren behandelt werden, beispielsweise Ätzen, Strahlen, Fräsen oder Drehen, um so eine bessere Haftung zur Elektrode zu erreichen. Die Elektrodenfixierhülse kann beispielsweise aus einem Harz, einem Polymer, einem Elastomer oder aus einem Thermoplast gegossen werden, wobei das Versteifungselement zumindest abschnittsweise mit dem Wandmaterial umgossen wird.

Im Folgenden ist die Erfindung anhand von beispielhaften Ausführungsformen mit Bezug auf die Zeichnungen näher erläutert. Die einzelnen Merkmale der Ausführungsformen können dabei beliebig miteinander kombiniert werden.

Es zeigen:
- Fig. 1: ein erstes Ausführungsbeispiel einer Elektrodenfixierhülse in einer Seitenansicht;
- Fig. 2: eine perspektivische Ansicht einer Elektrodenfixierhülse eines weiteren Ausführungsbeispiels der Figur 1 in einer durchscheinenden Ansicht;
- Fig. 3: eine perspektivische Darstellung eines Versteifungselementes der Elektrodenfixierhülse des Ausführungsbeispiels der Figur 2;
- Fig. 4: ein weiteres Ausführungsbeispiel der Elektrodenfixierhülse in einer durchscheinenden Ansicht;
- Fig. 5: eine perspektivische Ansicht eines Versteifungselementes des in der Figur 4 dargestellten Ausführungsbeispiels der Elektrodenfixierhülse;
- Fig. 6: ein weiteres Ausführungsbeispiel eines Versteifungselementes in einer perspektivischen Ansicht;
- Fig. 7: eine Schnittdarstellung einer Elektrodenfixierhülse mit einem Versteifungselement gemäß dem Ausführungsbeispiel der Figur 6;
- Fig. 8: ein weiteres Ausführungsbeispiel einer Elektrodenfixierhülse in einer Schnittdarstellung;
- Fig. 9: ein Versteifungselement in einer perspektivischen Ansicht gemäß dem Ausführungsbeispiel der Figur 8;
- Fig. 10: ein weiteres Ausführungsbeispiel einer Elektrodenfixierhülse in einer perspektivischen durchscheinenden Ansicht;
- Fig. 11: ein Versteifungselement gemäß dem Ausführungsbeispiel der Figur 10 in einer perspektivischen Ansicht;
- Fig. 12: ein weiteres Ausführungsbeispiel einer Elektrodenfixierhülse in einer perspektivischen durchscheinenden Ansicht und
- Fig. 13: ein Versteifungselement gemäß dem Ausführungsbeispiel der Figur 12 in einer perspektivischen Ansicht.

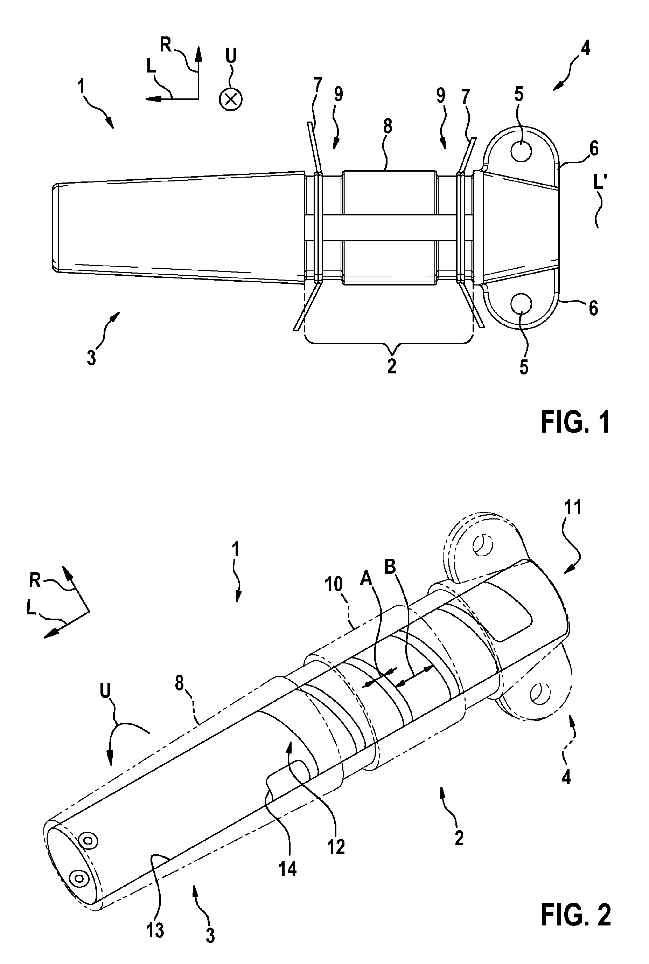

Zunächst sind Aufbau und Funktion einer erfindungsgemäßen Elektrodenfixierhülse mit Bezug auf das Ausführungsbeispiel der Figur 1 beschrieben.

Figur 1 zeigt eine Elektrodenfixierhülse 1 schematisch in einer Seitenansicht. Durch die Elektrodenfixierhülse 1 erstreckt sich entlang ihrer Längsrichtung L eine in der Seitenansicht der Figur 1 nicht sichtbare Aufnahmeröhre zur Aufnahme einer Elektrode. Die Aufnahmeröhre definiert ein vorzugsweise zylindrisch ausgebildetes Aufnahmevolumen für die Elektrode, wobei sich das Aufnahmevolumen entlang einer Längsachse L' der Elektrodenfixierhülse 1 erstreckt.

Die Elektrodenfixierhülse 1 ist mit einem Befestigungsabschnitt 2 ausgebildet, der entgegen einer quer zur Längsrichtung L verlaufenden Radialrichtung R auf die Längsachse L' zu verformbar ist. Wird der Befestigungsabschnitt 2 in Richtung auf die Längsachse L' verformt, so verringert sich ein Innendurchmesser der Aufnahmeröhre. Hierdurch wird eine kraftschlüssige Verbindung zwischen der Elektrodenfixierhülse 1 und der in die Aufnahmeröhre eingesetzten Elektrode hergestellt.

In der Längsrichtung L kann die Elektrodenfixierhülse 1 einen Führungsabschnitt 3 aufweisen, durch den sich ebenfalls die Aufnahmeröhre erstreckt und der die Elektrode führt. Im implantierten Zustand der Elektrodenfixierhülse 1 kann der Führungsabschnitt 3 zumindest teilweise in ein Gefäß hineinragen, durch das sich die Elektrode bis zu einem Organ, beispielsweise das Herz eines Patienten, erstrecken kann. Der Führungsabschnitt 3 ist also vorzugsweise so elastisch ausgebildet, dass er das Gefäß nicht unnötig verletzt.

In der Längsrichtung L vor dem Befestigungsabschnitt 2 kann die Elektrodenfixierhülse 1 einen Fixierabschnitt 4 aufweisen, der ausgebildet ist, um die Elektrodenfixierhülse 1 an einem Gewebeabschnitt des Patienten zu fixieren. Der Fixierabschnitt 4 kann wenigstens eine Fixieröffnung 5 aufweisen, durch die die Elektrodenfixierhülse 1 mit dem Gewebeabschnitt vernähbar ist. Die Fixieröffnung 5 erstreckt sich beispielsweise senkrecht zur Längsrichtung L durch einen sich von der Längsachse L' weg erstreckenden Fixierlappen 6. Insbesondere weist der Fixierabschnitt 4 zwei Fixieröffnungen 5 auf, die symmetrisch zur Längsachse L' angeordnet sind. Sind zwei Fixieröffnungen 5 vorgesehen, so können auch zwei Fixierlappen 6 ausgebildet sein, wobei je eine der Fixieröffnungen 5 sich durch einen der Fixierlappen 6 erstreckend angeordnet ist und wobei sich die beiden Fixierlappen 6 von der Längsachse L' und von einander weg erstrecken.

Um die kraftschlüssige Verbindung zwischen dem Befestigungsabschnitt 2 und der Elektrode herzustellen, werden entgegen der Radialrichtung R wirkende Befestigungskräfte auf den Befestigungsabschnitt 2 ausgeübt. Die Befestigungskräfte können beispielsweise durch einen Ligaturfaden 7 hervorgerufen werden, der um den Befestigungsabschnitt 2 herum zu binden ist. Da der Ligaturfaden 7 linienförmige Befestigungskräfte hervorruft, die durch ein Wandmaterial 8 des Befestigungsabschnitts 2 auf die Elektrode übertragen werden, droht bei einer zu großen und durch die linienförmigen Befestigungskräfte hervorgerufenen Belastung eine Beschädigung der Elektrode. Im in der Figur 1 gezeigten Ausführungsbeispiel werden zwei Ligaturfäden zur Erzeugung der Befestigungskräfte verwendet. Das Wandmaterial 8 des Befestigungsabschnitts 2 entspricht jedoch vorzugsweise dem Wandmaterial 8 des Führungsabschnitts 3. Da der Führungsabschnitt 3 zur Vermeidung unnötiger Verletzungen des Gefäßes weich ausgebildet ist, ist das Wandmaterial 8 im Bereich des Befestigungsabschnitts 2 bekannter Elektrodenfixierhülsen 1 ebenfalls weich und daher nicht in der Lage, die Befestigungskräfte großflächig zu verteilen, um eine Beschädigung der Elektrode zu verhindern. Folglich weist der Befestigungsschnitt 2 der erfindungsgemäßen Elektrodenfixierhülse 1 vorzugsweise eine größere Steifigkeit auf, als der Führungsabschnitt 3 und/oder der Fixierabschnitt 4.

Entlang seiner quer zur Längsrichtung L und zur Radialrichtung R verlaufenden Umfangsrichtung U der Elektrodenfixierhülse 1 kann sich eine von der Längsachse L' weg öffnende Befestigungsnut 9 erstrecken, die im Bereich des Befestigungsabschnitts 2 angeordnet ist. Die Befestigungsnut 9 verhindert, dass der Ligaturfaden 7 parallel zur Längsachse L' verrutscht, was ein Befestigen der Elektrodenfixierhülse 1 an der Elektrode erleichtert. Insbesondere kann der Befestigungsabschnitt 2 zwei Befestigungsnuten 9 aufweisen, die in der Längsrichtung L voneinander beabstandet angeordnet sind. Die Befestigungsnuten 9 können in der Längsrichtung L ein vorderes beziehungsweise ein hinteres Ende des Befestigungsabschnitts 2 ausbilden. Zusätzlich zur Haltefunktion der Befestigungsnut 9 ist eine Dicke des Wandmaterials 8 durch die Befestigungsnut 9 verringert, sodass die Befestigungskräfte einfacher durch die Elektrodenfixierhülse 1 auf die Elektrode übertragen werden.

Figur 2 zeigt ein weiteres Ausführungsbeispiel der erfindungsgemäßen Elektrodenfixierhülse 1 schematisch in einer durchscheinenden Perspektivdarstellung. Für Elemente, die in Funktion und/oder Aufbau den Elementen dem bisherigen Ausführungsbeispiel entsprechen, sind dieselben Bezugszeichen verwendet. Der Kürze halber ist lediglich auf die Unterschiede zum bisherigen Ausführungsbeispiel eingegangen.

Die Elektrodenfixierhülse 1 ist in der Figur 2 mit einem Versteifungselement 10 gezeigt, das im Bereich des Befestigungsabschnitts 2 angeordnet ist. Das Versteifungselement 10 erhöht die Steifigkeit des Befestigungsabschnitts 2, ohne dass das Wandmaterial 8 der Elektroden-fixierhülse 1 sich im Bereich des Befestigungsabschnitts 2 vom Wandmaterial 8 der restlichen Elektrodenfixierhülse 1 zu unterscheiden braucht. Ohne das Versteifungselement 10 wäre die Elektrodenfixierhülse 1 im Bereich des Befestigungsabschnitts 2 mit einem anderen Wandmaterial 8 als im Bereich des Führungsabschnitts 3 und/oder des Fixierabschnitts 4 auszubilden. Alternativ könnte sich die Wandstärke der Elektrodenfixierhülse 1 im Bereich des Befestigungsabschnitts 2 von der Wandstärke des Führungsabschnitts 3 und/oder von der Wandstärke des Fixierabschnitts 4 unterscheiden und insbesondere größer sein, um auftretende Befestigungskräfte besser verteilen zu können. Ein Vorsehen des Versteifungselementes 10 erleichtert jedoch die Fertigung der Elektrodenfixierhülse 1 und verhindert, dass die Elektrodenfixierhülse 1 im Bereich des Befestigungsabschnitts 2 unnötig verdickt ist. Wie im Ausführungsbeispiel der Figur 2 dargestellt, kann sich das Versteifungselement 10 in und/oder entgegen der Längsrichtung L über den Befestigungsabschnitt 2 hinaus erstrecken, um auch auf ein Ende oder einen Anfang des Befestigungsabschnitts 2 einwirkende Befestigungskräfte wirksam verteilen zu können.

Entgegen der Radialrichtung R ist das Versteifungselement 10 verformbar, sodass es die Befestigungskräfte auf die Elektrode übertragen kann. Vorzugsweise ist das Versteifungselement 10 entgegen der Radialrichtung R elastisch verformbar, sodass bei einer Verringerung der Befestigungskräfte die Verformung des Versteifungselementes 10 abnimmt und das Versteifungselement 10 zumindest teilweise in seinen unverformten Zustand zurückkehren kann. Hierdurch ist eine Entnahme oder ein nachträgliches Verschieben der Elektrode in/oder entgegen der Längsrichtung L vereinfacht. Die sich durch die Elektrodenfixierhülse 1 erstreckende Aufnahmeröhre 11 ist in der Figur 2 erkennbar. Die Aufnahmeröhre 11 erstreckt sich in der Längsrichtung L durch den Befestigungsabschnitt 2 sowie durch den Führungsabschnitt 3 und den Fixierabschnitt 4. Sie öffnet sich in und entgegen der Längsrichtung L, sodass die Elektrode durch die Aufnahmeröhre 11 hindurch schiebbar ist. Die Aufnahmeröhre 11 definiert ein Aufnahmevolumen, in das die Elektrode zumindest teilweise einsetzbar ist.

Wird die Elektrode in oder entgegen der Längsrichtung L in die Aufnahmeröhre 11 ein- oder durch diese hindurch geschoben, so besteht das Risiko, dass das Versteifungselement 10 parallel zur Längsrichtung L verschoben wird. Ist das Versteifungselement 10 zu weit aus dem Befestigungsabschnitt 2 herausgeschoben, so kann es die auftretenden Befestigungskräfte nicht mehr ausreichend verteilen, wodurch das Risiko einer Beschädigung der Elektrode vergrößert wäre. Um ein ungewolltes Verschieben des Versteifungselementes 10 zu verhindern, ist das Versteifungselement 10 vorzugsweise in das Wandmaterial 8 eingelassen. Beispielsweise kann das Versteifungselement 10 zumindest abschnittsweise mit dem Wandmaterial 8 umgossen sein und sogar daran haften.

Das Versteifungselement 10 ist also unverlierbar am Wandmaterial 8 befestigt und bildet einen integralen Bestandteil der Elektrodenfixierhülse 1. Die Elektrodenfixierhülse 1 mit dem Versteifungselement 10 ist folglich einstückig handhabbar, sodass das Versteifungselement 10 nicht separat zu handhaben ist, wenn die Elektrodenfixierhülse 1 implantiert werden soll.

Das Versteifungselement 10 kann an das durch die Aufnahmeröhre 11 definierte Aufnahmevolumen für die Elektrode angrenzen. Somit kann das Versteifungselement 10 an der Elektrode anliegen, wenn diese an der Elektrodenfixierhülse 1 befestigt ist. Ein an der Elektrode anliegendes Versteifungselement 10 erzeugt einen definierten Kontakt zwischen der Elektrodenfixierhülse 1 und der Elektrode, wodurch die Befestigungssicherheit von der Elektrodenfixierhülse 1 an der Elektrode verbessert ist.

Vorzugsweise fluchtet eine Innenseite 12 des Versteifungselementes 10 mit einer Innenseite 13 des Wandmaterials 8 zumindest im Bereich des Befestigungsabschnitts 2 und vorzugsweise auch im Bereich des Führungsabschnitts 3 und/oder des Fixierabschnitts 4, sodass sich die Aufnahmeröhre 11 zumindest im Bereich des Befestigungsabschnitts 2 stufenlos durch die Elektrodenfixierhülse 1 erstrecken kann. An Stufen oder Kanten kann die Elektrode beim Einsetzen in die Aufnahmeröhre 11 nämlich hängen bleiben, wodurch ein Einsetzen der Elektrode erschwert wäre.

Im Ausführungsbeispiel der Figur 2 ist das Versteifungselement 10 beispielhaft als eine Hohlwendel ausgebildet, deren zentrale Achse mit der Längsachse L' fluchtet. Das hohlwendelförmige Versteifungselement 10 windet sich um die Aufnahmeröhre 11 herum und weist gemäß dem dargestellten Ausführungsbeispiel fünf Windungen auf.

Das hohlwendelförmige Versteifungselement 10 ist durch eine gewundene Versteifungsplatte ausgebildet, deren parallel zur Längsrichtung L verlaufende Innenseite 12 größer ausgebildet ist, als deren quer zur Längsrichtung L verlaufende Seite 14. Entlang der Längsrichtung L kann das hohlwendelförmige Versteifungselement 10 einen rechteckigen Querschnitt aufweisen.

Ein sich parallel zur Längsrichtung L erstreckender Abstand A benachbarter Windungen des hohlwendelförmigen Versteifungselementes 10 ist vorzugsweise kleiner, als eine parallel zur Längsrichtung L zu messende Breite B der Versteifungsplatte. Durch den Abstand A ist eine gleichmäßige Verformbarkeit des hohlwendelförmigen Versteifungselementes 10 gewährleistet, wenn die Befestigungskräfte auf die Elektrodenfixierhülse 1 einwirken.

Wird die Elektrodenfixierhülse 1 in Folge der Befestigungskräfte entgegen der Radialrichtung R auf die Längsachse L' hin verformt, so verringert sich ein Innendurchmesser des hohlwendelförmigen Versteifungselementes 10 gleichmäßig entlang der Längsrichtung L, sodass punktuelle oder linienförmige Belastungen der Elektrode vermieden werden.

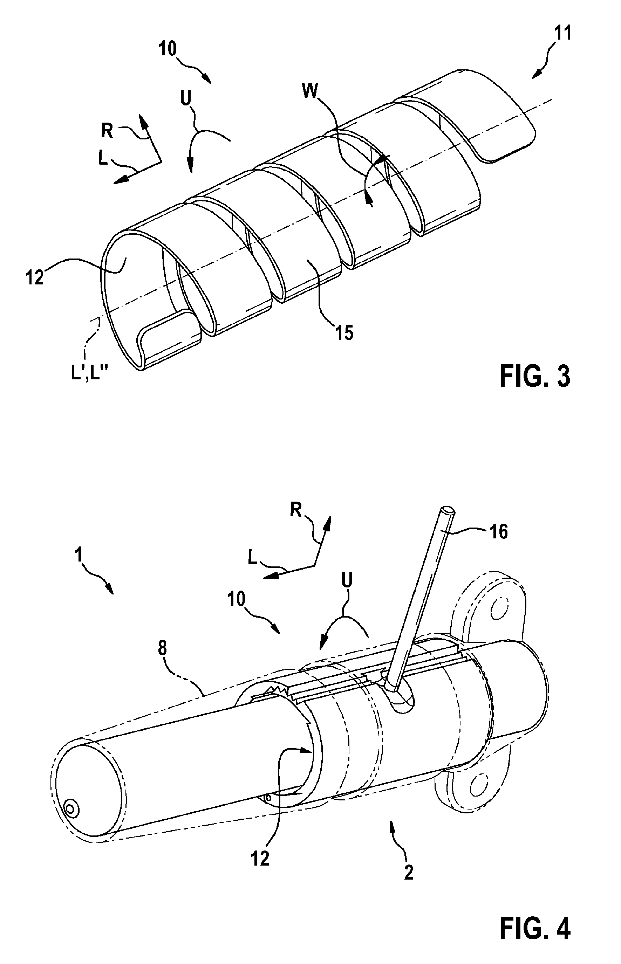

Figur 3 zeigt das hohlwendelförmige Versteifungselement 10 des Ausführungsbeispiels der Figur 2 schematisch.

Eine zentrale Längsachse L" des hohlwendelförmigen Versteifungselementes 10 fluchtet mit der Längsachse L' der Aufnahmeröhre 11. Um die Längsachse L' herum windet sich das hohlwendelförmige Versteifungselement 10. Um eine möglichst dichte Anlage zwischen dem hohlwendelförmigen Versteifungselement 10 und der Elektrode gewährleisten zu können, ist eine Steigung der Windungen des hohlwendelförmigen Versteifungselementes 10 so bemessen, dass ein Winkel W zwischen der Längsachse L" und den einzelnen Windungen möglichst groß ist. Insbesondere liegt der Winkel W zwischen 45° und 90° und beträgt beispielsweise 60°, 70°, 80° oder gar 85°.

Im Ausführungsbeispiel der Figur 3 ist gut erkennbar, dass die Innenseite 12 die Aufnahmeröhre 11 in der Radialrichtung R so begrenzt, dass die Aufnahmeröhre 11 zumindest im Bereich des hohlwendelförmigen Versteifungselementes 10 die Form eines geraden Zylinders haben kann. Die Innenseite 12 der Windungen des Versteifungselements 10 fluchten dabei genauso wie die Außenseite 15 der Windungen mit den anderen Windungen und verlaufen insbesondere parallel zur Längsachse L", sodass das hohlwendelförmige Versteifungselement 10 mit seiner Außenseite 15 und seiner Innenseite 12 einer zylindrischen Form folgt. Eine gleichmäßige Verformung der Elektrodenfixierhülse 1 im Bereich des Befestigungsabschnitts 2 führt also zu einer gleichmäßigen Weitergabe der Befestigungskräfte an die Elektrode.

Figur 4 zeigt ein weiteres Ausführungsbeispiel der erfindungsgemäßen Elektrodenfixierhülse 1 schematisch in einer durchscheinenden Perspektivansicht. Für Elemente, die in Funktion und/oder Aufbau den Elementen der bisherigen Ausführungsbeispiele entsprechen, sind dieselben Bezugszeichen verwendet. Der Kürze halber ist lediglich auf die Unterschiede zu den bisherigen Ausführungsbeispielen eingegangen.

Das Versteifungselement 10 des Ausführungsbeispiels der Figur 4 ist als eine Versteifungshülse ausgebildet, die zwei in beziehungsweise entgegen der Umfangsrichtung U relativ zueinander schwenkbare Teilschalen aufweist. Figur 4 zeigt ferner einen Öffnungsstift 16 zum Öffnen der Versteifungshülse. Das als Versteifungshülse ausgebildete Versteifungselement 10 ist wie das Versteifungselement 10 des Ausführungsbeispiels der Figuren 2 und 3 im Befestigungsabschnitt 2 angeordnet und kann in und entgegen der Längsrichtung L in den Führungsabschnitt 3 und/oder dem Fixierabschnitt 4 hineinragen. An einer Innenseite 12 des Versteifungselementes 10 kann Wandmaterial 8 vorgesehen sein, um ein direkten Kontakt eines Abschnittes des Versteifungselementes 10 mit der Elektrode zu verhindern. Insbesondere ein Einklemmen der Elektrode beim Zusammenklappen der Teilschalen kann hierdurch sicher verhindert werden.

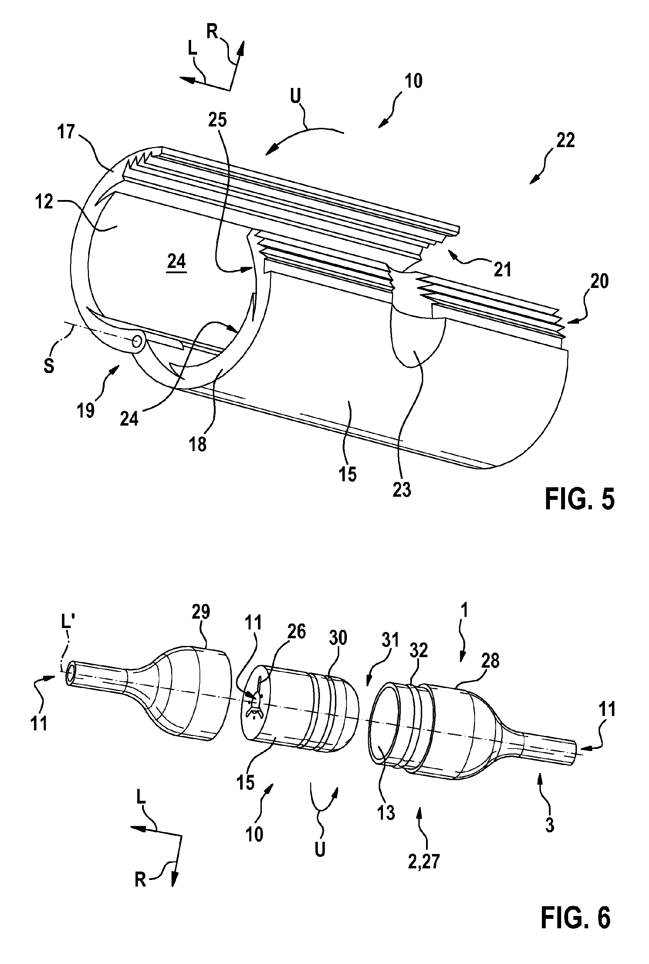

Weitere Details des als Versteifungshülse ausgebildeten Verstärkungselementes 10 sind in der Figur 5 dargestellt, die das als Versteifungshülse ausgestaltete Versteifungselement 10 des Ausführungsbeispiels schematisch in einer geöffneten Perspektivansicht zeigt.

Das Versteifungselement 10 des Ausführungsbeispiels der Figur 5 ist mit zwei Teilschalen 17, 18 ausgebildet, die um eine gemeinsame Schwenkachse S in beziehungsweise entgegen der Umfangsrichtung U relativ zueinander schwenkbar sind. Die Schwenkachse S verläuft vorzugsweise parallel und beabstandet zur Längsachse L".

Um schwenkbar miteinander verbunden zu sein, bilden die Teilschalen 17, 18 ein Scharnier 19 aus, dass die Lage der Schwenkachse S definiert. In beziehungsweise entgegen der Umfangsrichtung U dem Scharnier 19 gegenüberliegend angeordnete Endabschnitte der Teilschalen 17, 18 sind mit Rasteinrichtungen 20, 21 versehen, wobei die Rasteinrichtungen 20, 21 eine Rastvorrichtung 22 der Versteifungshülse ausbilden. Die Rastvorrichtung 22 befestigt die Teilschalen 17, 18 in einem geschlossenen Zustand aneinander, sodass die Versteifungshülse nicht ungewollt geöffnet werden kann.

Die Rasteinrichtung 20 der Teilschale 18 ist beispielsweise an der Außenseite 15 des Versteifungselementes 10 und die Rasteinrichtung 21 ist beispielsweise an der Innenseite 12 der Teilschale 17 angeordnet. Im geschlossenen Zustand des Versteifungselementes 10 weisen die Rasteinrichtungen 20, 21 aufeinander zu und liegen aneinander an. Beispielsweise können die Rasteinrichtungen 20, 21 jeweils Rastelemente oder Gegenrastelemente, etwa in Form von Rastzähnen oder -balken aufweisen, die ein Schließen des Versteifungselementes 10 durch ein Schwenken der Teilschalen 17, 18 parallel zur Umfangsrichtung U und aufeinander zu ermöglichen. Ein Schwenken der Teilschalen 17, 18 um die Schwenkachse S voneinander weg ist durch die Rastvorrichtung 22 im geschlossenen Zustand des Versteifungselementes 10 sicher verhindert.

Zum Lösen der Rastverbindung der Teilschalen 17, 18 miteinander kann insbesondere die Teilschale 18 entgegen der Radialrichtung R und in Richtung auf die Längsachse L' der Elektrodenfixierhülse 1 gedrückt werden. Weisen die Rasteinrichtungen 20, 21 Rastzähne beziehungsweise -balken auf, so sind diese im geschlossenen Zustand des Versteifungselementes 10 miteinander im Eingriff. Durch ein Verschieben der Rasteinrichtung 20 der Teilschale 18 entgegen der Radialrichtung R werden die Rastelemente der Rasteinrichtung 20 aus dem Eingriff mit den Gegenrastelementen der Rasteinrichtung 21 herausbewegt, sodass die Rastverbindung gelöst und das Versteifungselement 10 geöffnet werden kann. Zum Verformen der Teilschale 18 kann der Öffnungsstift 16 entgegen der Radialrichtung gegen die Teilschale 18 gedrückt werden.

Um ein Abrutschen des Öffnungsstiftes 16 von der Teilschale 18 zu verhindern, kann das Versteifungselement 10 im Bereich der Rasteinrichtung 20 eine Haltemulde 23 für den Öffnungsstift 16 aufweisen, die sich entgegen der Radialrichtung R in das Versteifungselement 10 hineinwölbt. Die Haltemulde 23 sichert den Öffnungsstiftes 16 gegen ein Verrutschen parallel zur Längsrichtung L beziehungsweise entgegen der Umfangsrichtung U. Im Wandmaterial 8 der Elektrodenfixierhülse 1 kann eine die Haltemulde 23 entgegen der Radialrichtung R zugänglich machende Öffnung für den Öffnungsstift 16 ausgeformt sein. Damit der Öffnungsstift 16 auch in einem nur teilweise geschlossenen Zustand des Versteifungselementes 10 sicher die Teilschale 18 verformen kann, ohne von dieser abzurutschen, kann sich die Haltemulde 23 in der Umfangrichtung U bis zwischen die Rastelemente der Rasteinrichtung 20 erstrecken.

Im Bereich des Scharniers 19 und der Rasteinrichtungen 20, 21 droht beim Schließen des als Versteifungshülse ausgebildeten Versteifungselementes 10 eine Beschädigung der Elektrode. Um die Elektrode beabstandet vom Scharnier 19 und der Rastvorrichtung 22 halten zu können, weist vorzugsweise wenigstens eine der Teilschalen 17, 18 einen in der Umfangsrichtung U zwischen der Rasteinrichtung 20, 21 und dem Scharnier 19 angeordneten Halteabschnitt 24 auf, der entgegen der Radialrichtung R und entlang der jeweiligen Teilschale 17, 18 das Scharnier S beziehungsweise die Rastvorrichtung 22 überragt. Folglich liegt die Elektrode nur im Bereich des wenigstens einen Halteabschnitts 24 an der Fixierhülse an. Der Halteabschnitt 24 kann an das Aufnahmevolumen angrenzen und somit die Elektrode direkt kontaktieren. Insbesondere das Scharnier 19 und eine entgegen der Rastrichtung R zum Scharnier 19 weisende Innenseite 25 der Rasteinrichtung 20 können durch Wandmaterial 8 von der Aufnahmeröhre 11 getrennt sein, sodass diese die Elektrode nicht direkt kontaktieren können.

Figur 6 zeigt ein weiteres Ausführungsbeispiel eines Versteifungselementes 10 für eine Elektrodenfixierhülse 1. Für Elemente, die in Funktion und/oder Aufbau den Elementen der bisherigen Ausführungsbeispiele entsprechen, sind dieselben Bezugszeichen verwendet. Der Kürze halber ist lediglich auf die Ausführungsbeispiele der bisherigen Figuren eingegangen.

Das Versteifungselement 10 des Ausführungsbeispiels der Figur 6 ist als ein Versteifungszylinder ausgebildet. Entlang der Längsachse L' erstreckt sich die Aufnahmeröhre 11 durch den Versteifungszylinder hindurch, wobei ein Innendurchmesser der Aufnahmeröhre 11 kleiner ist, als eine maximale Wandstärke des dickwandigen Versteifungszylinders in der Radialrichtung R. Um die Elektrode im Versteifungszylinder verklemmen und hierzu den Versteifungszylinder entgegen der Radialrichtung R zusammendrücken zu können, weist der Versteifungszylinder wenigstens eine Schwächungsnut 26 auf, die sich parallel zur Längsachse L erstreckt und entgegen der Radialrichtung R zur Aufnahmeröhre 11 hin öffnet. In der Radialrichtung R endet die Schwächungsnut 26, bevor sie die Außenseite 15 erreicht. Da ein Außendurchmesser quer zur Längsachse L' des als dickwandiger Versteifungszylinder ausgebildeten Versteifungselementes 10 deutlich größer ist, als ein Außendurchmesser des Führungsabschnittes 3, ist die Elektrodenfixierhülse 1 mit einem Aufnahmebehälter 27 ausgebildet, der den Befestigungsabschnitt 2 ausformt. Der Aufnahmebehälter 27 weist zwei Teilschalen 28, 29 auf, deren Innendurchmesser quer zur Längsachse L' größer ist, als der Innendurchmesser der Aufnahmeröhre 11, sodass das als dickwandiger Aufnahmezylinder ausgestaltete Versteifungselement 10 im Aufnahmebehälter 27 angeordnet werden kann.

In die Teilschale 28 kann das Versteifungselement 10 eingesetzt werden. Um das Versteifungselement 10 in der Teilschale 28 parallel zur Längsrichtung L zu fixieren, weist das Versteifungselement 10 an seiner Außenseite 15 wenigstens ein Rastelement, beispielsweise ein sich in der Umfangsrichtung U um das Versteifungselement 10 herum erstreckende Rastnut 30 auf. An der Innenseite 13 der Teilschale 28 ist ein Gegenrastelement für das Rastelement des Versteifungselementes 10 angeordnet, wobei das Gegenrastelement beispielsweise als ein Rastvorsprung oder ein sich entgegen der Radialrichtung R in den Aufnahmebehälter 27 hinein und in der Umfangsrichtung U um die Längsachse L' herum erstreckender Rastwulst ausgeformt sein kann.

Um das Versteifungselement 10 des Ausführungsbeispiels der Figur 6 einfach in die Teilschale 28 einsetzen zu können, ist ein der Längsrichtung L ausgebildetes Ende 31 verrundet ausgebildet.

Ist das Versteifungselement 10 in einer Montageposition in der Teilschale 28 angeordnet, so können das Versteifungselement 10 und die Teilschale 28 in der Längsrichtung L an eine gemeinsame Ebene angrenzen.

Die Teilschale 29 ist zumindest abschnittsweise in der Längsrichtung L auf die Teilschale 28 aufsetzbar. Die Teilschalen 28, 29 können ebenfalls über Rastelemente aneinander befestigbar sein. Beispielsweise weist die Teilschale 28 einen von der Längsachse L weg vorspringenden und sich in der Umfangsrichtung U um die Längsachse L' herum erstreckenden Aufnahmewulst 32 und die Teilschale 29 ein komplementäres Rastelement, dass an ihrer Innenseite angeordnet ist, auf.

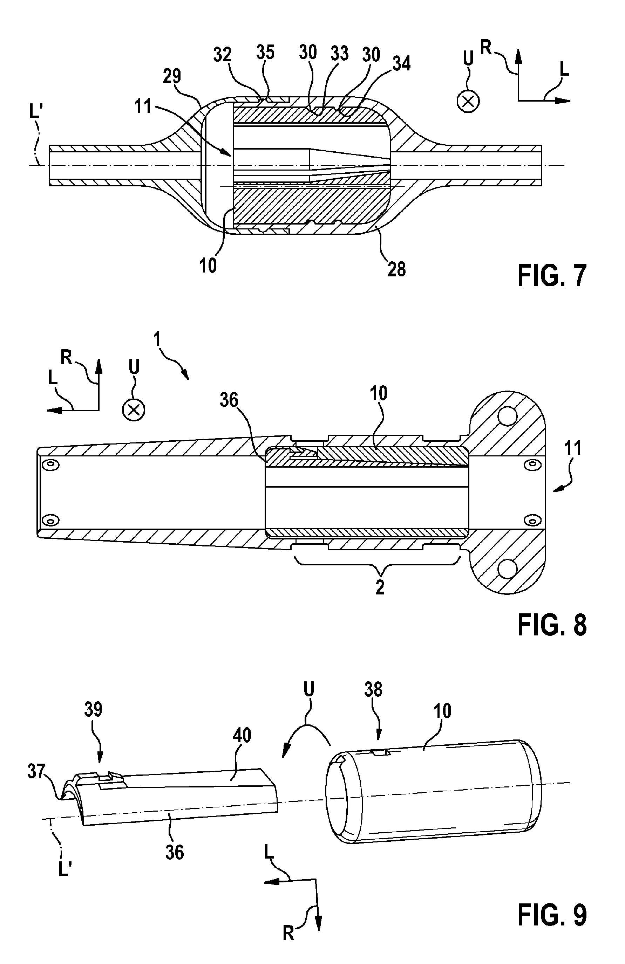

Figur 7 zeigt das Ausführungsbeispiel der Figur 6 schematisch in einer Schnittansicht, wobei die Schnittebene entlang der Längsachse L' verläuft. Das Versteifungselement 10 des Ausführungsbeispiels der Figur 7 ist mit zwei Rastnuten 30 versehen, die entlang der Längsrichtung L voneinander beabstandet sind. Dementsprechend weist die Teilschale 28 zwei auf die Längsachse L' zu vorspringende Rastwulste 33, 34 auf, welche im montierten Zustand des Versteifungselementes 10 in die Rastnuten 30 eingreifen und somit ein ungewolltes Verschieben des Versteifungselementes 10 parallel zur Längsrichtung L und relativ zur Teilschale 28 verhindern. Die Teilschalen 28, 29 sind ebenfalls über eine Rastverbindung gegen ungewollte Relativbewegungen entlang der Längsrichtung L gesichert. Die Rastverbindung ist auch hier durch eine Rastnut 32 und einen Rastwulst 35 gewährleistet.

Parallel zur Längsrichtung L ist das Versteifungselement 10 beabstandet von der Teilschale 29 angeordnet und mit der Teilschale 28 befestigt. Durch den Abstand zwischen dem Versteifungselement 10 und der Teilschale 29 ist eine Überbestimmtheit der Positionen der Teilschalen 28, 29 durch das Versteifungselement 10 verhindert, sodass die Teilschalen 28, 29 sicher durch Verrasten aneinander befestigbar sind.

Innerhalb des Versteifungselementes 10 kann sich die Aufnahmeröhre 11 in der Längsrichtung L zumindest abschnittsweise verjüngen, um die Elektrode gut führen zu können. Insbesondere wenn die Elektrode in der Längsrichtung L in die Aufnahmeröhre 11 eingesetzt werden soll, kann die Elektrode durch die sich verjüngende Aufnahmeröhre 11 gut positionierbar sein, da die Aufnahmeröhre 11 einen Trichter ausbildet.

Figur 8 zeigt ein weiteres Ausführungsbeispiel der erfindungsgemäßen Elektrodenfixierhülse 1 schematisch in einer Schnittdarstellung entlang der Längsachse L'. Für Elemente, die in Funktion und/oder Aufbau den Elementen der bisherigen Ausführungsbeispiele entsprechen, sind dieselben Bezugszeichen verwendet. Der Kürze halber ist lediglich auf die Unterschiede zu den bisherigen Ausführungsbeispielen eingegangen.

Die Elektrodenfixierhülse 1 ist mit einem zylinderförmigen Versteifungselement 10 versehen. Das Versteifungselement 10 ist als ein Hohlzylinder ausgebildet und kann zumindest entgegen der Radialrichtung R durch auftretende Befestigungskräfte im Wesentlichen unverformbar sein. Auch im in der Figur 8 gezeigten Ausführungsbeispiel erstreckt sich die Aufnahmeröhre 11 durch die Elektrodenfixierhülse 1 und das Versteifungselement 10.

Um die Elektrode in der Elektrodenfixierhülse 1 befestigen zu können, weist die Elektrodenfixierhülse 1 einen Klemmkeil 36 auf, der beispielsweise entgegen der Längsrichtung L in das zylinderförmige Versteifungselement 10 eingesetzt werden kann.

Details des Versteifungselementes 10 und des Klemmkeils 36 sind in der Figur 9 besser erkennbar, welche lediglich das zylinderförmige Versteifungselement 10 und den Klemmkeil 36 schematisch in einer Perspektivansicht zeigt.

Eine Innenseite 37 des Klemmkeils 36 ist im Wesentlichen bogenförmig und sich parallel zur Umfangsrichtung U erstreckend ausgebildet und liegt am Aufnahmevolumen der Aufnahmeröhre 11 an, wenn der Klemmkeil 36 entgegen der Längsrichtung L in das zylinderförmige Versteifungselement 10 eingesetzt ist.

Um den Klemmkeil 36 im zylinderförmigen Versteifungselement 10 fixieren zu können, kann das Versteifungselement 10 ein Rastelement 38 und der Klemmkeil 36 ein Gegenrastelement 39 aufweisen, wobei das Gegenrastelement 39 vorzugsweise an einer in der Radialrichtung R hinter der Innenseite 37 angeordneten Außenseite des Klemmkeils 36 vorgesehen ist. Entgegen der Längsrichtung L kann eine schräg zur Längsachse L' und insbesondere vom Gegenrastelement 39 weg verlaufende und sich dabei der Längsachse L annähernde Klemmschräge 40 vorgesehen sein, die den Klemmkeil 36 beim Einsetzen in das zylinderförmige Versteifungselement 10 entgegen der Radialrichtung R in Richtung auf die Längsachse L' zu drückt und somit den Innendurchmesser der Aufnahmeröhre 11 verringert und dadurch die Elektrode großflächig verpresst.

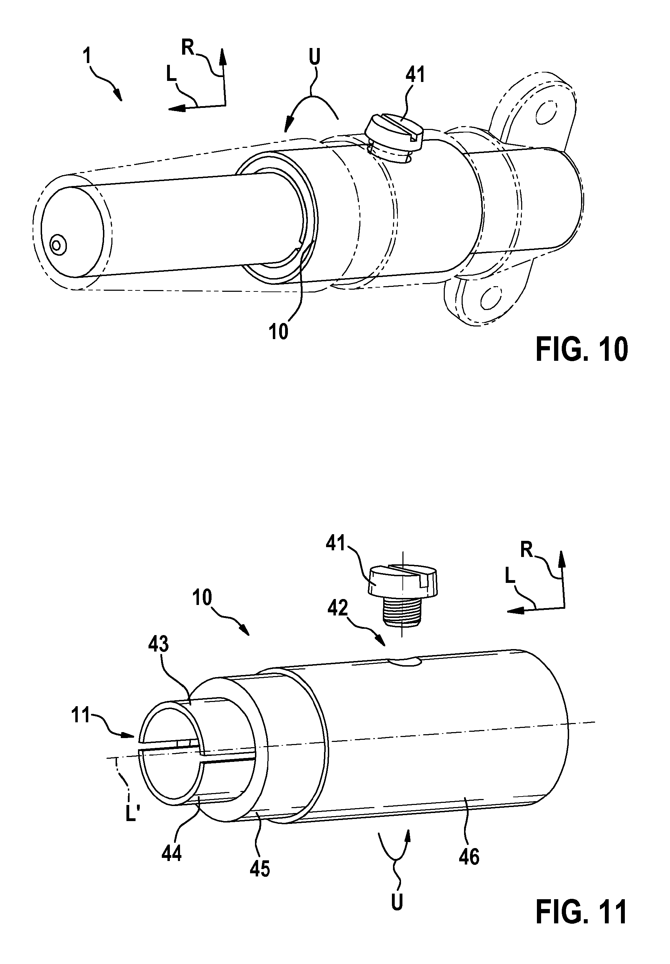

Figur 10 zeigt ein weiteres Ausführungsbeispiel der erfindungsgemäßen Elektrodenfixierhülse 1 schematisch in einer durchscheinenden Ansicht. Für Elemente, die in Funktion und/oder Aufbau den Elementen der Ausführungsbeispiele der bisherigen Figuren entsprechen, sind dieselben Bezugszeichen verwendet. Der Kürze halber ist lediglich auf die Unterschiede zu den bisherigen Ausführungsbeispielen eingegangen.

Auch im Ausführungsbeispiel der Figur 10 ist das Versteifungselement 10 zylinderförmig ausgebildet. Jedoch benötigt das zylinderförmige Versteifungselement 10 des Ausführungsbeispiels der Figur 10 keinen Klemmkeil. Vielmehr erstreckt sich in der Radialrichtung R eine Aufnahmeöffnung für ein Klemmelement 41, das entgegen der Radialrichtung R eine Befestigungskraft auf die Elektrode ausüben kann.

Weitere Details des zylinderförmigen Versteifungselements 10 sind in der Figur 11 besser ersichtlich, welche lediglich das zylinderförmige Versteifungselement 10 und das Klemmelement 41 schematisch in einer Perspektivansicht darstellt.

Das Klemmelement 41 kann als eine Schraube ausgebildet sein, die in die in der Figur 11 mit dem Bezugszeichen 42 versehene Aufnahmeöffnung entgegen der Radialrichtung R einsetzbar ist.

Bei einem direkten Kontakt des Klemmelementes 41 mit der Elektrode, droht eine mechanische Überlastung und eine folgende Beschädigung der Elektrode. Um eine solche Überlastung der Elektrode zu vermeiden, weist das Versteifungselement 10 zumindest eine innere Teilschale auf, welche durch das Klemmelement 41 entgegen der Radialrichtung R in Richtung auf die Längsachse L' verschiebbar ist. Die innere Teilschale 43 verteilt durch das Klemmelement 41 aufgebrachte Befestigungskräfte gleichmäßig auf zumindest eine Seite der Elektrode.

Das Versteifungselement 10 kann zumindest eine weitere innere Teilschale 44 aufweisen, die quer zur Längsrichtung L der inneren Teilschale 43 gegenüberliegend angeordnet ist, sodass sich die Aufnahmeröhre 11 durch die inneren Teilschalen 43, 44 erstrecken, welche an das durch die Aufnahmeröhre 11 definierte Aufnahmevolumen für die Elektrode angrenzen.

Die inneren Teilschalen 33, 34 können an einen parallel zur Radialrichtung R verlaufenden Halteschlauch 45 befestigt sein, welcher die inneren Teilschalen 33, 34 in einem geöffneten Zustand hält, wenn das Klemmelement 41 nicht in die Aufnahmeöffnung 42 eingesetzt ist. Das Klemmelement 41 kann zum Zusammendrücken der Teilschalen 43, 44 entweder direkt auf die innere Teilschale 43 oder auf den Halteschlauch 45 drücken. Der Halteschlauch 45 ist dabei vorzugsweise elastisch verformbar, sodass sich die Halteschalen 43, 44 voneinander entfernen, wenn das Klemmelement 41 aus der Öffnung 42 herausgenommen wird. Der Halteschlauch 45 ist also vorzugsweise in der Radialrichtung R zwischen den inneren Teilschalen 43, 44 und einer Außenhülse 46 des Versteifungselementes 10 angeordnet und sichert die Teilschalen 43, 44 gegen Verschiebungen entlang der Längsrichtung und relativ zur Aufnahmeöffnung 42.

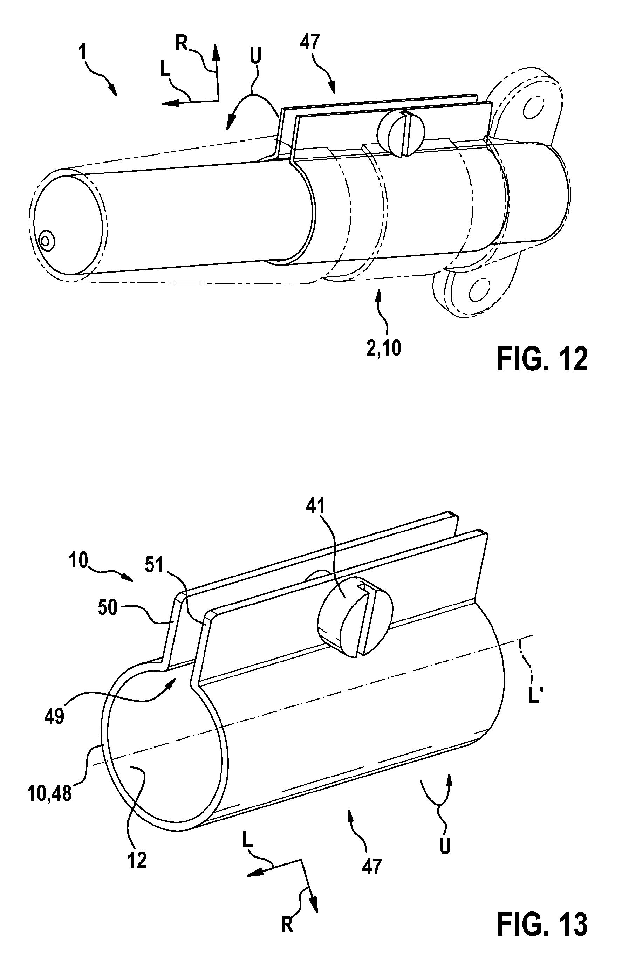

Figur 12 zeigt ein weiteres Ausführungsbeispiel eines eine Klemmeinrichtung aufweisenden Versteifungselements 10 der Elektrodenfixierhülse 1 schematisch in einer durchscheinenden Perspektivdarstellung. Für Elemente, die in Funktion und/oder Aufbau den Elementen der in den bisherigen Figuren gezeigten Ausführungsbeispiele entsprechen, sind dieselben Bezugszeichen verwendet. Der Kürze halber ist im Folgenden lediglich auf die Unterschiede zu den bisherigen Ausführungsbeispielen eingegangen.

Figur 12 zeigt die Elektrodenfixierhülse 1 mit einer Klemmeinrichtung 47 zur Befestigung der Elektrode, wobei die Klemmeinrichtung 47 zum Teil das Versteifungselement 10 ausbildet und in das Wandmaterial 8 der Elektrodenfixierhülse 1 eingebettet ist.

Weitere Details der Klemmeinrichtung 47 sind in der Figur 13 besser ersichtlich, da die Figur 13 lediglich die Klemmeinrichtung 47 in einer schematischen Perspektivdarstellung zeigt.

Die Klemmeinrichtung 47 ist mit einem Klemmabschnitt 48 ausgebildet, mit dem die Elektrode quer zur Längsrichtung L in der Elektrodenfixierhülse 1 kraftschlüssig gehalten werden kann. Der Klemmabschnitt 48 bildet gleichzeitig das Versteifungselement 10, da der Klemmabschnitt 48 auf die Klemmeinrichtung 47 einwirkende Befestigungskräfte gleichmäßig auf die Elektrode verteilt. Das Versteifungselement 10 kann also ganz allgemein für alle Ausführungsbeispiele auch als ein Kraftverteilungselement bezeichnet werden.

Der Klemmabschnitt 48 ist im Wesentlichen hohlzylindrisch ausgebildet und erstreckt sich in der Längsrichtung L. Der hohlzylinderförmige Klemmabschnitt 48 weist einen sich in der Längsrichtung L erstreckenden Spalt 49 auf. Aufgrund des Spaltes 49 können einander gegenüberliegend angeordnete Seiten des Klemmabschnittes 48 aufeinander zu gedrückt werden.

Zum Einbringen der Befestigungskraft und zum Zusammendrücken des Klemmabschnittes 48 kann das Versteifungselement 10 zwei Kraftaufnahmeabschnitte 50, 51 aufweisen, welche von in der Umfangsrichtung U einander gegenüberliegenden Enden des Klemmabschnitts 48 in der Radialrichtung R parallel zueinander vorspringen. Zwischen den Enden des Klemmabschnitts 48 erstreckt sich der Spalt 49. In die Kraftaufnahmeabschnitte 50, 51 wird die Befestigungskraft eingeleitet. Hierzu können die Kontaktaufnahmeabschnitte 50, 51 beispielsweise jeweils eine Öffnung aufweisen, wobei eine der Öffnungen mit einem Innengewinde ausgestaltet ist. Wird nun das Klemmelement 41, beispielsweise eine Schraube, in die Öffnungen eingesetzt, so kann das Befestigungselement 41 die Aufnahmeabschnitte 50, 51 aufeinander zu ziehen, wodurch die einander gegenüberliegend angeordneten Seiten des Klemmabschnitts 48 aufeinander zu bewegt und der Innendurchmesser der Aufnahmeröhre 11 verringert wird.

Zumindest der Klemmabschnitt 48 ist in das Wandmaterial 8 eingebettet und grenzt mit seiner Innenseite 12 an das durch die Aufnahmeröhre 11 definierte Aufnahmevolumen für die Elektrode an. Die Kraftaufnahmeabschnitte 50, 51 ragen vorzugsweise von der Längsachse L' aus dem Wandmaterial 8 heraus, um besser zugänglich zu sein.

## Patentansprüche

1. Elektrodenfixierhülse (1) zur Fixierung einer Elektrode an einem Gewebeabschnitt eines Patienten, mit einem Befestigungsabschnitt (2) zur Befestigung der Elektrodenfixierhülse (1) an der Elektrode, wobei die Elektrodenfixierhülse (1) eine sich in ihrer Längsrichtung (L) durch den Befestigungsabschnitt (2) erstreckende Aufnahmeröhre (11) für die Elektrode ausbildet und entgegen einer senkrecht zur Längsrichtung (L) verlaufenden Radialrichtung (R) zusammendrückbar ausgebildet ist, **dadurch gekennzeichnet, dass** der Befestigungsabschnitt (2) eine größere Steifigkeit aufweist, als ein benachbarter Abschnitt (3, 4) der Elektrodenfixierhülse (1).

2. Elektrodenfixierhülse (1) nach Anspruch 1, **dadurch gekennzeichnet, dass** die Elektrodenfixierhülse (1) ein Versteifungselement (10) aufweist, das im Befestigungsabschnitt (2) angeordnet ist und das eine größere Steifigkeit aufweist, als ein Wandmaterial (8) der Elektrodenfixierhülse (1), wobei das Versteifungselement (10) ein integraler Bestandteil der Elektrodenfixierhülse (1) ist.

3. Elektrodenfixierhülse (1) nach Anspruch 2, **dadurch gekennzeichnet, dass** das Versteifungselement (10) entgegen der Radialrichtung (R) verformbar ist.

4. Elektrodenfixierhülse (1) nach Anspruch 2 oder 3, **dadurch gekennzeichnet, dass** das Versteifungselement (10) in das Wandmaterial (8) eingelassen ist.

5. Elektrodenfixierhülse (1) nach den Ansprüchen 2 bis 4 **dadurch gekennzeichnet, dass** das Versteifungselement (10) an ein durch die Aufnahmeröhre (11) definiertes Aufnahmevolumen für die Elektrode angrenzt.

6. Elektrodenfixierhülse (1) nach einem der Ansprüche 2 bis 5, **dadurch gekennzeichnet, dass** eine Innenseite (12) des Versteifungselementes (10) mit einer Innenseite (13) des Befestigungsabschnitts (2) fluchtet.

7. Elektrodenfixierhülse (1) nach einem der Ansprüche 2 bis 6, **dadurch gekennzeichnet, dass** das Versteifungselement (10) als eine Hohlwendel ausgebildet ist.

8. Elektrodenfixierhülse (1) nach Anspruch 7, **dadurch gekennzeichnet, dass** die Hohlwendel eine gewundene Versteifungsplatte aufweist, deren parallel zur Längsrichtung (L) verlaufende Seiten (12, 15) größer sind, als deren quer zur Längsrichtung (L) verlaufende Seiten.

9. Elektrodenfixierhülse (1) nach Anspruch 8, **dadurch gekennzeichnet, dass** ein parallel zur Längsrichtung (L) bemessener Abstand (A) benachbarter Windungen der Hohlwendel kleiner ist, als eine parallel zur Längsrichtung (L) bemessene Breite (B) der Versteifungsplatte.

10. Elektrodenfixierhülse (1) nach einem der Ansprüche 7 bis 9, **dadurch gekennzeichnet, dass** die Hohlwendel wenigstens eine Windung aufweist.

11. Elektrodenfixierhülse (1) nach einem der Ansprüche 7 bis 10, **dadurch gekennzeichnet, dass** die Hohlwendel eine ganzzahlige Anzahl an Windungen aufweist.

12. Verfahren zum Herstellen einer Elektrodenfixierhülse (1) zur Fixierung einer Elektrode an einem Gewebeabschnitt eines Patienten, mit einem Befestigungsabschnitt (2) zur Befestigung der Elektrodenfixierhülse (1) an der Elektrode, wobei die Elektrodenfixierhülse (1) mit einer sich in ihrer Längsrichtung (L) durch den Befestigungsabschnitt (2) erstreckenden Aufnahmeröhre (11) für die Elektrode und der Befestigungsabschnitt (2) entgegen einer senkrecht zur Längsrichtung (L) verlaufenden Radialrichtung (R) zusammendrückbar ausgeformt wird, **dadurch gekennzeichnet, dass** der Befestigungsabschnitt (2) mit einer größeren Steifigkeit ausgeformt wird, als ein benachbarter Abschnitt (3, 4) der Elektrodenfixierhülse (1).

13. Verfahren nach Anspruch 12, **dadurch gekennzeichnet, dass** ein Versteifungselement (10) in den Befestigungsabschnitt (2) integriert wird.

14. Verfahren nach Anspruch 13, **dadurch gekennzeichnet, dass** das Versteifungselement (10) in ein Wandmaterial (8) des Befestigungsabschnitts (2) eingebettet wird.

15. Verfahren nach Anspruch 13 oder 14, **dadurch gekennzeichnet, dass** das Versteifungselement (10) zumindest abschnittsweise mit dem Wandmaterial (8) umgossen wird.
